# EUROPEAN PATENT SPECIFICATION

(11) **EP 2 451 810 B1**
(45) Date of publication and mention of the grant of the patent: **20.11.2013**
(21) Application number: 10747301.9
(22) Date of filing: 07.07.2010
(51) Int. Cl.: C07D 471/04

(54) **CONTINUOUS FLOW PROCESS FOR THE PREPARATION OF SULPHOXIDE COMPOUNDS**
DURCHLAUFVERFAHREN ZUR HERSTELLUNG VON SULFOXID VERBINDUNGEN
PROCÉDÉ A FLUX CONTINU POUR LA PRÉPARATION DE COMPOSÉS SULFOXIDES

(30) Priority: 07.07.2009 IN DE13922009
(43) Date of publication of application: 16.05.2012
(73) Proprietor: Council of Scientific & Industrial Research, New Delhi 110 001 (IN)
(72) Inventor: KULKARNI, Amol, Arvind, Maharashtra (IN); JOSHI, Ramesh, Anna, Maharashtra (IN); NIVANGUNE, Nayana, Tushar, Maharashtra (IN); JAGTAP, Manisha, Abdhiman, Maharashtra (IN)
(74) Representative: Thomson, Craig Richard
(86) International application number: PCT/IN2010/000456
(87) International publication number: WO 2012/004802

(56) References cited:
- US-A1- 2006 089 376
- TAKUYA NOGUCHI, YOSHIRO HIRAI AND MASAYUKI KIRIHARA: "Highly selective 30% hydrogen peroxideoxidation of sulfides to sulfoxides using micromixing" CHEMICAL COMMUNICATIONS, 2008, pages 3040-3042, XP002602693 DOI: 10.1039/B802502A cited in the application

## Description

### Technical field of the invention:

The invention relates to a continuous process for the preparation of sulphoxide compounds. Particularly the invention relates to an efficient micromixer based continuous flow process for the synthesis of sulphoxide compounds such as modafinil compounds and proton pump inhibitors, with a high degree of selectivity.

### Background and prior art:

Sulphinyl compounds predominantly find use as proton pump inhibitors such as pantaprazole, rabeprazole, lansoprazole, as modafinil compounds and such like. The total world-wide sale of esomprazole, a proton pump inhibitor was over 6 billion in 2006.

Batch processes for the synthesis of these compounds are described in prior art documents.

US 4808596 discloses a process for the synthesis of sulfinyl compounds by reacting the starting compound in chloroform with m-perchlorobenzoic acid at 0 to -5 degree C. The process of the invention is to be carried out at -70 to -30 °C, preferably -20 to 10 ° C for a period of time ranging approximately from 1 minute to 24 hours, preferably from 5 minutes to 1 hour.

"Highly selective 30% hydrogen peroxide oxidation of sulfides to sulfoxides using micromixing" by Takuya Noguchi, Chem. Commun., 2008, 3040 - 3042, US2008108122 and "Investigation of Micromixing Efficiency in a Novel High-Throughput Microporous Tube-in-Tube Microchannel Reactor" by Qi-An Wang, Jie-Xin Wang, et al Ind. Eng. Chem. Res., 2009, 48 (10), pp 5004-5009 present processes for the conversion of sulphides to sulphoxides by various methods.

US 7439367 relates to a batch process for the preparation of a sulfinyl compound involving the oxidation of a sulfide compound in the presence of a catalyst wherein the oxidising agents are aqueous alkali or alkali earth metal hypohalite solution. JP1190682, US2006089376, WO2006024890 and WO2008/087665 also disclose batch processes for the preparation of prazole type compounds.

But the prior art processes suffer from drawback such as maintenance of low temperature conditions leading to long processing time. The low temperature maintenance is required to prevent the formation of unnecessary side products and thus improve the specificity of the reaction and its conversion rate. The process of the 367 patent claims about 85% yield of the desired product over a duration of 1 - 4 hours. Therefore there is a need in the art to provide for a more efficient process for the synthesis of sulphinyl compounds.

Further there is a need in the art to have a process for the synthesis of sulphinyl compounds which is quick and does not consume time.

Also, there is a need in the art to provide for a more efficient and quick process for the synthesis of sulphinyl compounds with high selectivity to sulfoxide compounds which in turn results in high yield and purity of the desired product with lower impurities of sulfones.

Further the efficient and quick process for the synthesis of sulphinyl compounds should have high degree of specificity towards the formation of desired sulphoxide compounds.

There is also a need in the industrial level to provide an efficient and quick process for the synthesis of sulphinyl compounds which leads to very low yields of unnecessary side products such as sulfones in a continuous manner as against such features only in batch processing.

### Summary of the invention:

The instant invention discloses a continuous process for the synthesis of sulphoxide compounds wherein the process isconducted in a T -shaped micromixer that results in reaction time of 1 minute. The oxidizing agent of the invention is preferably m-chloroperbenzoic acid.. The reaction results in over 90% conversion and > 95% selectivity towards sulphoxide compounds with lesser than 5% formation of undesired sulfones.

### Description of drawing:

Figure 1: Schematic of the experimental set-up with the two syringes to inject the two reactants connected to the micromixer followed by a single inlet microreactor.
Figure 2: Schematic presentation of the experimental set-up with the microreactor with spatially discretely located inlets with micromixers at different distances for multipoint injection of one of the reactants.

### Detailed description of the invention:

In accordance with the above objectives of the invention, a micromixer based, continuous process for the synthesis of sulphoxide compounds with a high degree of selectivity of > 95% towards sulphoxide compounds at temperature range of -5 - 50 °C, in less than or equal to a minute is disclosed herein. The continuous process for the synthesis of sulphoxide of formula 1 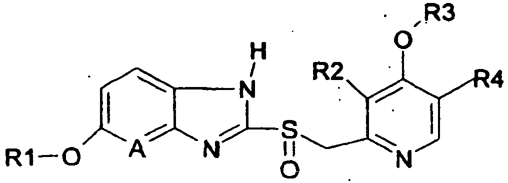 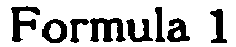 compounds comprises:
a. mixing imidazo[4,5-b]pyridine compound of formula 2 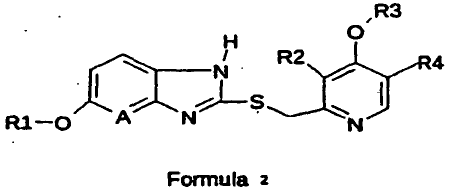 with an oxidizing agent dissolved in a solvent, in a T-shaped micromixer with a reaction tube;
b. maintaining the temperature of the reaction tube at -5 - 50 °C and specifically in the range of 5 - 25 °C for about a minute and
c. isolating the product of the process.

The imidazo[4,5-b]pyridine compound of formula 1 is as shown herein 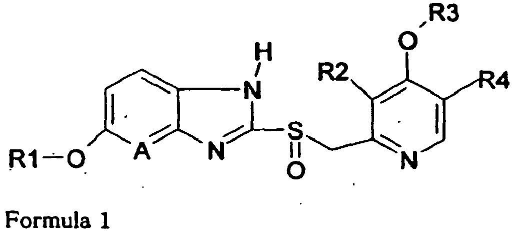 wherein A is carbon or nitrogen and R1, R2. R3 , R4 are alkyl groups. In one embodiment of the invention, R1, R2, R3 and R4 are same. In another embodiment of the invention R1, R2, R3 and R4 are different.

The oxidizing agent of the invention is preferably m-chloroperbenzoic acid(m-CPBA). The oxidizing agent is used in the concentration range of 0.5 - 20 equimolar ratio. The solvents are selected from chloroform and methanol, alone or in combinations thereof. In combination, the solvents are used in the ratio of 0 - 0.5, v/v. The concentration of substrate required for the reaction ranges from 0.01 - 0.1 w/v.

The product obtained from the internally structured T-shaped micromixer with the reaction tube within a reaction time of less than or equal to one minute is with sulfone content not greater than 5% and selectivity of > 95% towards the desired sulphoxide compounds. The process of the invention results in greater than 90% conversion of reactant to respective sulphoxide compound with yield of sulphoxide compound greater than 90%.

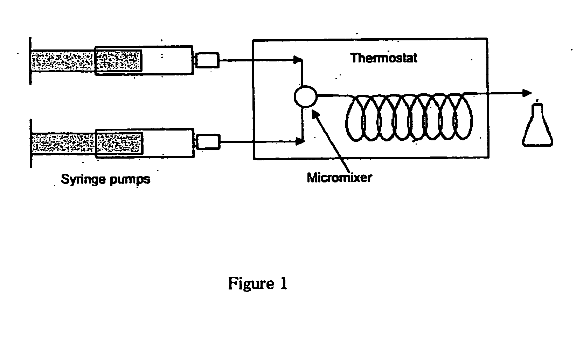

With reference to figure 1, depicting the single inlet microreactor for the continuous flow experiments, the experimental set-up consisted of two syringe pumps loaded with glass syringes connected to SS316 tubes [1/16" (1.58mm) o.d.] through an in-house developed and fabricated glass to metal connector made in PTFE. The two metallic tubes were subsequently connected to a micromixer followed by a residence time tube [1/16" (1.58mm) o.d.] which was immersed in a thermostat. The tube can be made of SS316 or Hastelloy. Syringes were filled with each of the reactants and the flow rates were set to achieve the desired residence time in the reaction tube.

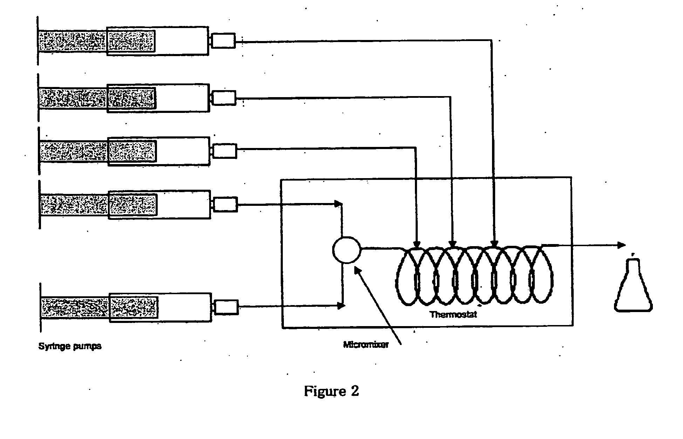

In another embodiment of the invention, the continuous process of the invention is carried out by using a 1 m long tube with spatially discretely located multi point inlets. The number of inlets vary in the range of 2 to 6 and the discrete inlets (schematic shown in Figure 2) are maintained at equal spacing. While the imidazo[4,5-b]pyridine compound is injected at the first inlet, the other inlets are used for injecting the oxidizing agent either with equal flow rates or at different flow rates depending up on the need to vary the residence time and the concentration. In yet another embodiment of the invention, the inlets are maintained at unequal spacing.

The reaction of the invention is schematically represented herein:

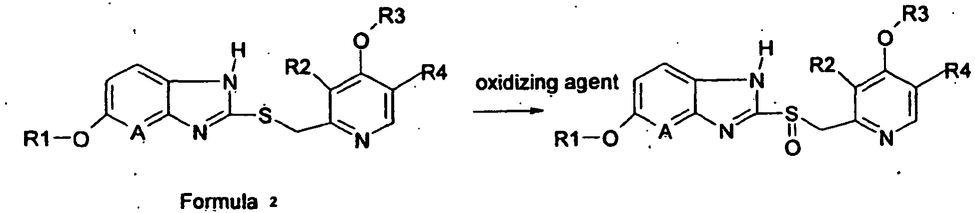

Wherein A is carbon or nitrogen and R1, R2, R3, R4 are alkyl groups. In one embodiment of the invention, R1, R2, R3 and R4 are same. In another embodiment of the invention R1, R2, R3 and R4 are different.

The sulphoxide compounds particularly are proton pump inhibitors such as omeprazole, pantoprazole, lansoproazole, tenatoprazole, rabeprazole and modafinil compounds.

The process of the invention has the following advantages:
1. It is capable of being easily scaled-up.
2. The process has provided the choice of solvent other than only chloroform, a volatile solvent, since use of chloroform alone changes the concentration of the reaction mass as it evaporates at room temperature.
3. The process is continuous with minimal reaction time of less than or equal to one minute.
4. The sulfone content formation is less than 5%, resulting in high yield of sulphoxide compounds with high selectivity of > 95% towards sulphoxide compounds.
5. The conversion rate is greater than 90%.

### Examples:

### Example 1: Experimental set up for examples 1-10

For the continuous flow experiments typically, the experimental set-up involved two syringe pumps (Boading Longer, China) followed by a micromixer, which was then' connected to a 1 m long stainless steel (SS316) tube [1/16" (1.58mm) o.d. and 1.38mm i.d.]. The SS tube was immersed in a thermostat (Julabo - ME12, Germany) and the samples were collected at the outlet of the tube. The residence time was varied by changing the flow rates. The samples were collected in alkali solution to quench the reaction at the outlet of the reaction tube. The product was subjected to analysis after further dilution. The general reaction scheme for all examples described is depicted herein. The imidazo[4,5-b]pyridine compound of Formula 3 as shown herein was used for the purpose of exemplification of the present invention.

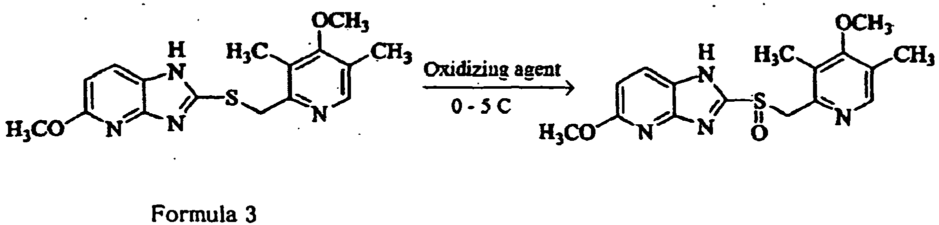

### Example 2:

100 mg of imidazo[4,5-b]pyridine compound of formula 3 was dissolved in 10 ml chloroform and 80 mg of H₂O₂ in 10 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 3% conversion to sulphoxide compound.

### Example 3:

100 mg of imidazo[4,5-b]pyridine compound of formula 3 was dissolved in 10 ml chloroform and 80 mg of sodium hypochlorite in 10 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 2% conversion to sulphoxide compound.

### Example 4:

100 mg of imidazo[4,5-b]pyridine compound of Formula 3 was dissolved in 10 ml chloroform and 60 mg of m-CPBA in 10 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 82% conversion to sulphoxide compound. The selectivity towards sulphoxide compound was 94% and 6% sulphone was formed.

### Example 5:

100 mg of imidazo[4,5-b]pyridine compound of Formula 3 was dissolved in 10 ml chloroform and 70 mg of m-CPBA in 10 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 85% conversion to sulphoxide compound. The selectivity towards sulphoxide compound was 93% and 7% sulphone was formed.

### Example 6:

100 mg of imidazo[4,5-b]pyridine compound of Formula 3 was dissolved in 10 ml chloroform and 87 mg of m-CPBA in 10 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 97% conversion to sulphoxide compound. The selectivity towards sulphoxide compound was 92% and 8 % sulphone was formed.

### Example 7:

200 mg of imidazo[4,5-b]pyridine compound of Formula 3 was dissolved in 20 ml chloroform and 160 mg of m-CPBA in 20 ml solvent chloroform. The two reacting solutions were mixed using a T micro mixer followed by a 1m long retention time tube. The process was carried out at 0 ° C. The residence time in the tube was maintained at 60 seconds. The analysis of product formed showed 96% conversion to sulphoxide compound. The selectivity towards sulphoxide compound was 96% and 4 % sulphone was formed.

### Example 8:

200 mg of imidazo[4,5-b]pyridine compound of Formula 3 was dissolved in 20 ml chloroform and 160 mg of m-CPBA in 20 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 96% conversion to sulphoxide compound. The selectivity towards sulphoxide compound was 96% and 4 % sulphone was formed.

### Example 9:

100 mg of imidazo[4,5-b]pyridine compound of Formula 3 was dissolved in 10 ml chloroform and 100mg of m-CPBA in 10 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 90% conversion to sulphoxide compound. The selectivity towards sulphoxide compound was 95% and 5 % sulphone was formed.

### Example 10:

100 mg of imidazo[4,5-b]pyridine compound of Formula 3 was dissolved in 10 ml methanol and 80mg of m-CPBA in 5 ml solvent chloroform. The two reacting solutions were mixed using a T micro mixer followed by a 1m long retention time tube. The process was carried out at 5 ° C. The residence time in the tube was maintained at 60 seconds. The analysis of product formed showed 98% conversion to sulphoxide compound. The selectivity towards sulphoxide compound was 95% and 5 % sulphone was formed.

### Example 11:

100 mg of imidazo[4,5-b]pyridine compound of Formula 3 was dissolved in 10 ml of equal volume of chloroform and methanol and 80mg of m-CPBA in 5 ml equal volume of chloroform and methanol. The two reacting solutions were mixed using a T micro mixer followed by a 1m long retention time tube. The process was carried out at 5 °C. The residence time in the tube was maintained at 60 seconds. The analysis of product formed showed 98% conversion to sulphoxide compound. The selectivity towards sulphoxide compound was 95% and 5 % sulphone was formed.

### Example 12:

Examples 11-14 were in the multipoint micromixer with spatially discretely located inlets. 100 mg of imidazo[4,5-b]pyridine compound of Formula 3 was dissolved in 10 ml chloroform and 80mg of m-CPBA in 10 ml solvent chloroform. The microreactor was built with multipoint inlets for the m-CPBA. The solution of imidazo[4,5-b]pyridine compound of Formula 3 dissolved in chloroform was injected continuous at the first inlet of the reactor while the solution of m-CPBA was injected continuously at different proportions through the four inlets located discretely along the reactor length for a 1m long retention time tube. At every inlet a T micro mixer was used for inline mixing. The overall residence time of the reaction mixture was maintained at 60 s and at 5 °C reaction temperature. The analysis of product formed showed 85% conversion to sulphoxide compound. The selectivity towards sulphoxide compound was 99% and 0.5 % sulphone was formed.

### Example 13:

For the composition of reacting solutions as given in Example 11, experiments with 50 s residence time and at 5 °C reaction temperature yielded 97% conversion to sulphoxide compound. The reduction in residence time was achieved by increasing the flow rate of m-CPBA in all the four inlets along the length of reactor. The selectivity towards sulphoxide compound was 98.5% and 1 % sulphone was formed.

### Example 14:

For the composition of reacting solutions as given in Example 11, experiments with 60 s residence time and at 15 °C reaction temperature yielded 90% conversion to sulphoxide compound. The reduction in residence time was achieved by increasing the flow rate of m-CPBA in all the four inlets along the length of reactor. The selectivity towards sulphoxide compound was 99% and 1 % sulphone was formed.

### Example 15:

For the reaction conditions given in the Example 12 and at 15 °C reaction temperature, the multipoint reactor yielded 98% conversion to sulphoxide compound. The reduction in residence time was achieved by increasing the flow rate of m-CPBA in all the four inlets along the length of reactor. The selectivity towards sulphoxide compound was 98% and sulphone was formed in the range of less than 1.5%.

## Claims

1. A continuous micromixer based process for the synthesis of sulphoxide compounds of formula 1 with a reaction time of less than or equal to one minute comprising:
a) mixing imidazo[4,5-b]pyridine compound of formula 2 with an oxidizing agent dissolved in a solvent in a T-shape micromixer with a reaction tube;
b) maintaining the temperature of the reaction tube at -5 to 50 °C and;
c) isolating the product, wherein the selectivity of the process towards the sulphoxide compounds is > 95%,
wherein formula 1 is 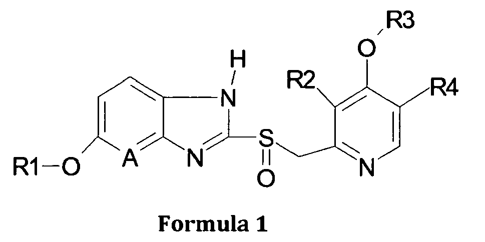 wherein A is carbon or nitrogen and R1, R2, R3, R4 are alkyl groups
and wherein formula 2 is 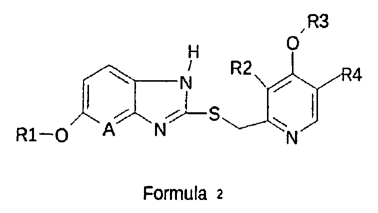 wherein A is carbon or nitrogen and R1, R2, R3, R4 are alkyl groups

2. The process as claimed in claim 1, wherein said oxidizing agent is m chloroperbenzoic acid in 0.5 - 20 equimolar ratio.

3. The process as claimed in claim 1, wherein said solvent is selected from chloroform, methanol and combinations thereof.

4. The process as claimed in claim 1, wherein said solvents are in the ratio of 0 0.5v/v.

5. The process as claimed in claim 1, wherein said imidazo[4,5-b]pyridine compounds are in the range of 0.01- 0.1 w/v.

6. The process as claimed in claim 1, wherein the micromixer comprises a single point inlet.

7. The process as claimed in claim 1, wherein the micromixer comprises a multi point inlet.

8. The process as claimed in claim 1, wherein the temperature of the reaction tube is maintained in the range of 5 to 25°C.

## Patentansprüche

1. Ein kontinuierliches, mikromischerbasiertes Verfahren für die Synthese von Sulfoxidverbindungen der Formel 1 mit einer Reaktionszeit von weniger als oder gleich einer Minute, das Folgendes beinhaltet:
a) Mischen einer Imidazo[4,5-b]pyridinverbindung der Formel 2 mit einem in einem Lösungsmittel aufgelösten Oxidationsmittel in einem T-förmigen Mikromischer mit einem Reaktionsgefäß;
b) Halten der Temperatur des Reaktionsgefäßes bei-5 bis 50 °C; und
c) Isolieren des Produktes, wobei die Selektivität des Verfahrens zu den Sulfoxidverbindungen > 95 % beträgt,
wobei Formel 1 folgende ist: 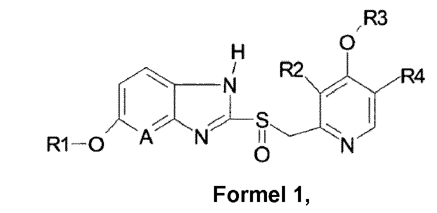 wobei A Kohlenstoff oder Stickstoff ist und R1, R2, R3, R4 Alkylgruppen sind, und wobei Formel 2 folgende ist: 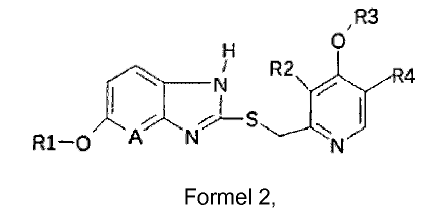 wobei A Kohlenstoff oder Stickstoff ist und R1, R2, R3, R4 Alkylgruppen sind.

2. Verfahren gemäß Anspruch 1, wobei das Oxidationsmittel m-Clorperbenzoesäure in einem äquimolaren Verhältnis von 0,5-20 ist.

3. Verfahren gemäß Anspruch 1, wobei das Lösungsmittel aus Chloroform, Methanol und Kombinationen davon ausgewählt ist.

4. Verfahren gemäß Anspruch 1, wobei die Lösungsmittel in dem Verhältnis von 0 0,5 Volumenanteil vorliegen.

5. Verfahren gemäß Anspruch 1, wobei die Imidazo[4,5-b]pyridinverbindungen in dem Bereich von 0,01-0,1 Massenkonzentration vorliegen.

6. Verfahren gemäß Anspruch 1, wobei der Mikromischer einen Einpunkt-Einlass beinhaltet.

7. Verfahren gemäß Anspruch 1, wobei der Mikromischer einen Mehrpunkt-Einlass beinhaltet.

8. Verfahren gemäß Anspruch 1, wobei die Temperatur des Reaktionsgefäßes in dem Bereich von 5 bis 25 °C gehalten wird.

## Revendications

1. Un procédé en continu basé sur un micromélangeur pour la synthèse de composés sulfoxydes de formule 1 avec un temps de réaction inférieur ou égal à une minute comprenant le fait :
a) de mélanger un composé imidazo[4,5-b]pyridine de formule 2 avec un agent oxydant dissous dans un solvant dans un micromélangeur en forme de T avec un tube de réaction ;
b) de maintenir la température du tube de réaction à une température allant de -5 à 50 °C et ;
c) d'isoler le produit, dans lequel la sélectivité du procédé envers les composés sulfoxydes est > 95 %, dans lequel la formule 1 est 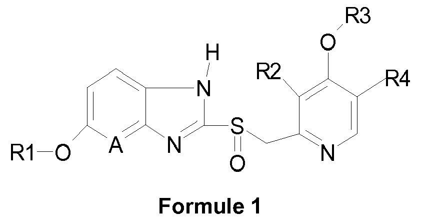 dans laquelle A est du carbone ou de l'azote et R1, R2, R3, R4 sont des groupes alkyles
et dans lequel la formule 2 est 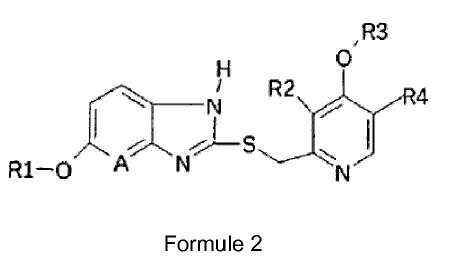 dans laquelle A est du carbone ou de l'azote et R1, R2, R3, R4 sont des groupes alkyles

2. Le procédé tel que revendiqué dans la revendication 1, dans lequel ledit agent d'oxydation est de l'acide m-chloroperbenzoïque dans un rapport équimolaire allant de 0,5 à 20.

3. Le procédé tel que revendiqué dans la revendication 1, dans lequel ledit solvant est sélectionné parmi le chloroforme, le méthanol et des combinaisons de ceux-ci.

4. Le procédé tel que revendiqué dans la revendication 1, dans lequel lesdits solvants sont dans le rapport de 0 0,5 v/v.

5. Le procédé tel que revendiqué dans la revendication 1, dans lequel lesdits composés imidazo[4,5-b]pyridine sont dans la gamme allant de 0,01 à 0,1 m/v.

6. Le procédé tel que revendiqué dans la revendication 1, dans lequel le micromélangeur comprend un seul point d'entrée.

7. Le procédé tel que revendiqué dans la revendication 1, dans lequel le micromélangeur comprend un point d'entrée multiple.

8. Le procédé tel que revendiqué dans la revendication 1, dans lequel la température du tube de réaction est maintenue dans la gamme allant de 5 à 25 °C.
